# EUROPÄISCHE PATENTSCHRIFT

(11) **EP 0 906 113 B1**
(45) Veröffentlichungstag und Bekanntmachung des Hinweises auf die Patenterteilung: **17.07.2002**
(21) Anmeldenummer: 96942233.6
(22) Anmeldetag: 23.12.1996
(51) Int. Cl.: A61K 35/78, A61K 9/48

(54) **VERFAHREN ZUM HERSTELLEN EINES LAGERFÄHIGEN PRÄPARATS AUS FRISCHPFLANZEN UND FRISCHPFLANZENPRÄPARAT**
PROCESS FOR PREPARING A STORABLE PREPARATION CONSISTING OF FRESH PLANTS, AND FRESH PLANT PREPARATION
PROCEDE POUR PRODUIRE UNE PREPARATION STOCKABLE A PARTIR DE PLANTES FRAICHES ET PREPARATION A BASE DE PLANTES FRAICHES AINSI OBTENUE

(30) Priorität: 14.03.1996 CH 67296
(43) Veröffentlichungstag der Anmeldung: 07.04.1999
(73) Patentinhaber: Greither, Peter, 9533 Kirchberg (CH)
(72) Erfinder: Greither, Peter, 9533 Kirchberg (CH)
(74) Vertreter: Wenger, René
(86) Internationale Anmeldenummer: CH9600463
(87) Internationale Veröffentlichungsnummer: WO9733596

(56) Entgegenhaltungen:
- EP-A- 0 496 705

## Beschreibung

Die Erfindung betrifft ein Verfahren zum Herstellen eines lagerfähigen Präparats aus Frischpflanzen.

Die Herstellung lagerfähiger Arzneimittel oder Genussmittel aus Pflanzen ist seit Jahrhunderten bekannt. Die in den Pflanzen enthaltenen Wirkstoffe werden dabei in der Form der getrockneten und gemahlenen Pflanzenmaterie (Droge), der flüssigen oder getrockneten Extrakte mit Wasser, Alkohol oder organischen Lösungsmitteln als Extraktionsmittel (Fluidextrakte, Spissumextrakte), roh aufgetrennt oder rein eingesetzt. Die Herstellung solcher Extrakte ist im wesentlichen dadurch geprägt, dass die frische Pflanze zunächst in eine lagerfähige, trockene Droge überführt wird, die zum gewünschten Zeitpunkt zum Extrakt der gewünschten Art weiterverarbeitet werden kann.

Ein Nachteil dieser altbekannten Verfahren besteht jedoch darin, dass die derart aus getrockneter Droge hergestellten Pflanzenauszüge, auch solche, die mit Wasser rehydriert werden, von der Art und Menge der darin enthaltenen Wirkstoffe nicht vollständig vergleichbar sind mit den Inhaltsstoffen der frischen Pflanzen. In der Regel finden beim Trocknen der Droge irreversible Fermentationen, Hydrolysevorgänge, Kondensationen, Oxidationen oder Verluste an flüchtigen Bestandteilen, sowie andere Veränderungen statt.

Durch die EP-A-496 705 ist eine aus frischen und/oder getrockneten Pflanzen gewonnene Masse bekannt geworden, die sich für die Einkapselung in Gelatine-Kapseln eignet. Das Ausgangsmaterial bildet dabei ein Teil- oder Vollextrakt, der auf übliche Art erhalten wird und der wenigstens ein Extraktionsmittel enthält. Beim Lösungs- oder Extraktionsmittel kann es sich um Methanol, Ethanol oder einfach um Wasser handeln. Durch den Extraktionsprozess erleiden die Inhaltsstoffe der Pflanzen jedoch teilweise ebenfalls irreversibele Veränderungen, so dass die Eigenschaften der verkapselbaren Masse nicht mehr mit denjenigen der frischen Pflanzen vergleichbar sind.

Seit langem bekannt sind selbstverständlich auch Verfahren zur Gewinnung von Präparaten aus frischen Pflanzen oder Pflanzenteilen, insbesondere von essbaren Früchten, wie z.B. Holundersaft oder Saft aus schwarzen Johannisbeeren und dergleichen. In der Regel führt jedoch die Herstellung von reinen Frischpflanzensäften zu Problemen während der Haltbarkeitsfrist durch Veränderung von Inhaltsstoffen durch Enzyme und andere Proteine, sowie durch Polysaccharide und Schleimstoffe, die während der Verarbeitung mitgewonnen werden. Im Gegensatz zur frischen, intakten Pflanze, in der empfindliche Stoffe sehr oft in verschiedenen Zellkompartimenten oder -organellen liegen, sind auch biochemische Umsetzungen während der Lagerdauer von Frischpflanzenpressaft möglich. Dies ist bei Fruchtsäften, die lediglich Genusszwecken dienen eher weniger problematisch, kann bei Arzneimitteln auf der Basis frischer Pflanzen jedoch nicht toleriert werden.

Es ist daher eine Aufgabe der Erfindung, ein Verfahren der eingangs genannten Art zu schaffen, mit dessen Hilfe die in der nativen frischen Pflanze enthaltenen Wirk- oder Geschmacksstoffe in eine lagerfähige Darreichungsform umgeformt werden können, ohne dass die in dieser Darreichungsform erwünschten Stoffe dabei wesentlich in der qualitativen und quantitativen Zusammensetzung von den in der frischen Pflanze vorhandenen Substanzen zur Zeit der Ernte abweichen. Gleichzeitig sollen die unerwünschten Stoffe, wie z.B. Schleimstoffe, ausfallende Proteine und Gerbstoffe eliminiert werden.

Diese Aufgabe wird erfindungsgemäss dadurch gelöst, dass aus erntefrischen Pflanzenteilen durch Pressen ein Frischpflanzensaft gewonnen wird, der zur Zerstörung der Enzymaktivität einer Kurzzeiterhitzung unterworfen wird, dass dem Frischpflanzensaft zur Matrixbildung bis 20% Mono- und/oder Disaccharide zugegeben wird, dass der Frischpflanzensaft anschliessend getrocknet und pulverisiert wird, und dass das Frischpflanzensaftpulver in einer hydrophoben Matrix in Kapseln, insbesondere Weichgelatinekapseln verkapselt wird. Es hat sich überraschend gezeigt, dass dieses schonende lösungsmittelfreie Verfahren lagerfähige Präparate mit dem unveränderten Substanzspektrum der frischen Pflanze liefert. Bei der Weichgelatinekapsel handelt es sich um eine wasserarme galenische Darreichungsform, deren Herstellungsverfahren an sich bereits gut beherrscht wird und die keine unerwünschten Reaktionen mit dem Frischpflanzenpräparat verursacht.

Bei der Herstellung des Frischpflanzenpressaftes aus den erntefrischen Pflanzenteilen findet vorteilhaft eine Zerkleinerung unter Schutzgas, eine sofortige Denaturierung der Proteine, sowie eine Keimzahlreduktion durch Uperisation statt. Dabei wird die Pflanze innert 10 bis max. 20 Stunden nach der Ernte verarbeitet und die Grobzerkleinerung findet unter relativ kühlen Bedingungen bei 10 bis 20° C und bei hoher Luftfeuchte bei 70 bis 100% rel. H statt. Die Pürierung der Pflanzenmasse findet unter Schutzgas, beispielsweise Stickstoff statt und der vollständige Aufschluss der Pflanzenzellen erfolgt vorzugsweise durch Vakuumextrusion unter hohem Vakuum von weniger als 10 mbar. Dagegen erfolgt die Dekantierung bei Ueberdruck von 1 bis 3 atm. über Filter gepresst, wobei der Rohsaft auf ca. 10° C gekühlt wird. Innerhalb von weniger als 120 Minuten wird der Rohsaft uperisiert (UHT-Verfahren) und durch eine dreistufige Filtration geklärt. Die Trocknung des Frischpflanzensaftes erfolgt dann vorteilhaft innerhalb von 24 Stunden nach der Gewinnung, spätestens aber innerhalb von 48 Stunden nach der Pressung.

Der Zusatz von Rosmarinextrakt als Antioxidans hat sich besonders bewährt, namentlich Rosmarinextrakt aus überkritischer Kohlendioxidextraktion.

Durch das Verfahren wird verhindert, dass eine Wirkstoffveränderung durch Oxidation, Hydrolyse oder durch bakteriologische Einwirkungen stattfinden kann.

Die Trocknung des Frischpflanzensaftes kann vorteilhaft in einem Vakuumbandtrockner erfolgen und zwar bei einer Temperatur von weniger als 50° C und bei einem reduzierten Druck von 6 bis 15 mbar. Vakuumbandtrockenverfahren sind dem Fachmann grundsätzlich bekannt und werden daher hier nicht näher beschrieben. Der Zusatz von bis zu 20% Mono- und/oder Disacchariden, insbesondere von Maltodextrin oder Lactose im Vakuumbandverfahren ergibt jedoch besonders kompakte, glasartig amorphe Strukturen, die sich durch ausgeprägte Stabilisierung der Wirkstoffstrukturen auszeichnen.

Zum Kaschieren des teilweise ureigenen Geschmackes der Frischpflanzenkonzentrate können zusätzliche Geschmacks- und Geruchskorrigentien in das Frischpflanzensaftpulver oder in die fertigen Darreichungsformen eingearbeitet werden. Ebenso können zum Erzielen synergistischer Effekte zusätzlich Vitamin- und/oder Mineralien und/oder Spurenelemente und/oder Provitamine eingearbeitet werden. Auch die Einarbeitung zusätzlicher pharmakologischer Wirkstoffe auf synthetischer oder natürlicher Basis, rein oder in Form von Extrakten oder Konzentraten kann in bestimmten Fällen sinnvoll sein.

Das vor dem Verkapseln anfallende Zwischenprodukt, also das Frischpflanzensaftpulver eignet sich auch sehr gut für die Verarbeitung zu anderen wasserarmen Darreichungsformen, insbesondere zur Herstellung eines Brausepräparats in Tablettenform oder als Granulat. Ebenso könnte das in die hydrophobe Matrix eingebettete Frischpflanzensaftpulver für einen Verbrauch in grösseren Mengen, beispielsweise auch in Tuben, Dosen, Beutel oder in anderen nicht für den Verzehr bestimmten Behältnissen eingefüllt werden. An die Stelle von Weichgelatinekapseln könnten in bestimmten Fällen auch Hartkapseln, insbesondere solche aus Stärke oder aus Cellulose-Derivaten treten. Die Verarbeitung zu Weichgelatinekapseln erfolgt auf besonders rationelle und einfache Weise nach dem Rotary-Die-Verfahren.

Für die Gelatinemasse wird vorteilhaft ein Gelatinetyp niedriger Viskosität mit einer niedrigen Bloom-Zahl verwendet, die eine wenig wasserhaltige Gelatineschmelze ergibt. Möglichst wasserfreie, hydrophobe Matrizes werden erzielt durch die Verwendung von langkettigen Trigylzeriden und Zusatz von Wachsen.

Grundsätzlich lassen sich alle pressbaren Pflanzenteile (Herba, Radix, Flos, Fructus, Cortex) auf die erfindungsgemässe Art zu Frischpflanzensaftpulver verarbeiten, wobei es sich um Heilpflanzen, Gewürzpflanzen oder auch um solche Pflanzen handeln kann, die normalerweise lediglich zu Genuss- oder Nahrungsmitteln verarbeitet werden. Die Verarbeitung und Verkapselung kann auch zu Aromazwecken erfolgen. Bei Arzneimitteln ist es je nach Indikation sinnvoll, die Kapselhülle durch geeignete Massnahmen magensaftresistent auszubilden.

Im pharmazeutischen Bereich wurden bei verschiedenen Indikationen besonders gute Resultate mit der Verarbeitung folgender Heilpflanzen erzielt:

| | |
|---|---|
| Acerola | Malpighia punicifolia |
| Artischocke | Cynara scolymus |
| Bärlauch | Allium ursinum |
| Baldrian | Valeriana officinalis |
| Birke | Betula Arten i.B. B. pendula |
| | B. pubescens |
| Bohne | Phaseolus vulgaris |
| Brennessel | Urtica dioica |
| Brunnenkresse | Nasthurtium officinale |
| Fenchel | Foeniculum vulgare |
| Gänsefingerkraut | Potentilla anserina |
| Ginkgo | Ginkgo biloba |
| Ginseng | Panax ginseng C.A. Meyer |
| Hafer | Avena sativa |
| Huflattich | Tussilago farfara |
| Johanniskraut | Hypericum perforatum |
| Kamille | Matricaria chamomilla |
| Knoblauch | Allium sativum |
| Kürbis | Cucurbita Arten i.B. C. pepo varietäten |
| Löwenzahn | Taraxacum officinale |
| Meerrettich | Armoracia rusticana |
| Melisse | Melissa officinalis |
| Mistel | Viscum album |
| Petersilie | Petroselium sativum |
| Rosmarin | Rosmarinus officinalis |
| Salbei | Salvia officinalis |
| Schafgarbe | Achillea millefolium |
| Schwarzrettich | Raphanus sativus varietäten |
| Sellerie | Apium graveolens |
| Sonnenhut | Echinacea angustifolia/purpurea |
| Spargel | Asparagus officinalis |
| Spitzwegerich | Plantago lanceolata |
| Teufelskralle | Harpagophytum procumbens |
| Thymian | Thymus vulgaris |
| Wacholder | Juniperus communis |
| Weissdorn | Crataegus laevigata/oxyacantha |
| Wermut | Artemisia absinthium |
| Zinnkraut | Equisetum arvense |

Ein paar Ausführungsbeispiele der Erfindung sind nachstehend wiedergegeben:
- Beispiel 1:: 200 mg Frischpflanzensaftpulver (hergestellt aus Frischpflanze Echinacea angustifolia und/oder Echinacea pallida und/oder Echinacea purpurea unter Zusatz von 20 bis 40 mg Dextrin und Lactose und 0,1 mg Rosmarinextrakt im Vakuumbandtrockner bei weniger als 50° C Temperatur und 6 bis 15 mbar Druck getrocknet) eingearbeitet in Weichgelatinekapseln mit einer Matrix aus Sojaöl, Sojaöl hydriert und Bienenwachs zu einer Kapselgrösse Format 7,5 minims.
- Beispiel 2:: Gleich wie Beispiel 1 aber mit einem Zusatz von 200 mg Vitamin C in Weichgelatinekapseln Format 16 minims.
- Beispiel 3:: 200 mg Frischpflanzensaftpulver (hergestellt aus Frischpflanze Artischokenblätter und Früchte unter Zusatz von bis 40 mg Glucose und 0,1 mg Rosmarinextrakt im Vakuumbandtrockner getrocknet bei weniger als 50° C Temperatur und 6 bis 15 mbar Druck), eingearbeitet in Weichgelatinekapseln mit einer Matrix aus Sojaöl, Sojaöl hydriert und Bienenwachs zu einer Kapselgrösse Format 7,5 minims.
- Beispiel 4:: 200 mg Frischpflanzensaftpulver (hergestellt aus Frischpflanze Ginkgoblätter unter Zusatz von 10 bis 20 mg Lactose und 0,1 mg Rosmarinextrakt im Vakuumbandtrockner getrocknet bei weniger als 50° C Temperatur und 6 bis 15 mbar Druck), eingearbeitet in Weichgelatinekapseln mit einer Matrix aus Sojaöl, Sojaöl hydriert und Bienenwachs zu einer Kapselgrösse von Format 7,5 minims.
- Beispiel 5:: 200 mg Frischpflanzensaftpulver (hergestellt aus Frischpflanze Weissdornblätter, Blüten und/oder Früchte unter Zusatz von 10 bis 20 mg Dextrin, Lactose und Rosmarinextrakt im Vakuumbandtrockner getrocknet bei weniger als 50° C Temperatur und 6 bis 15 mbar Druck), zusammen mit 36 IU Tocopherol eingearbeitet in Weichgelatinekapseln mit einer Matrix aus Sojaöl, Sojaöl hydriert und Bienenwachs zu einer Kapselgrösse Format 7,5 minims.

## Patentansprüche

1. Verfahren zum Herstellen eines lagerfähigen Präparats aus Frischpflanzen, **dadurch gekennzeichnet,**
- **dass** aus erntefrischen Pflanzenteilen durch Pressen ein Frischpflanzensaft gewonnen wird, der zur Zerstörung der Enzymaktivität einer Kurzzeiterhitzung unterworfen wird,
- **dass** dem Frischpflanzensaft zur Matrixbildung bis zu 20% Mono- und/oder Disaccharide zugesetzt wird,
- **dass** der Frischpflanzensaft anschliessend getrocknet und pulverisiert wird,
- und **dass** das Frischpflanzensaftpulver in einer hydrophoben Matrix in Kapseln, insbesondere Weichgelatinekapseln verkapselt wird.

2. Verfahren nach Anspruch 1, **dadurch gekennzeichnet, dass** die erntefrischen Pflanzenteile durch eine Vakuumextrusion aufgeschlossen werden.

3. Verfahren nach Anspruch 1 oder 2, **dadurch gekennzeichnet, dass** dem Frischpflanzensaft als Antioxidans Rosmarinextrakt zugesetzt wird.

4. Verfahren nach einem der Ansprüche 1 bis 3, **dadurch gekennzeichnet, dass** die Trocknung des Frischpflanzensaftes im Vakuumbandtrockenverfahren bei weniger als 50° C Temperatur und bei 6 bis 15 mbar Druck erfolgt.

5. Verfahren nach einem der Ansprüche 1 bis 4, **dadurch gekennzeichnet, dass** als hydrophobe Matrix langkettige Triglyzeride und Zusatz von Wachsen verwendet wird.

6. Lagerfähiges Präparat aus Frischpflanzen, insbesondere hergestellt nach dem Verfahren nach Anspruch 1, **gekennzeichnet durch** eine Weichgelatinekapsel mit einer Füllung aus Frischpflanzensaftpulver, das in einer hydrophoben Matrix eingebettet ist.

7. Präparat nach Anspruch 6, **gekennzeichnet durch** zusätzliche Geschmacks- und Geruchskorrigentien.

8. Präparat nach Anspruch 6 oder 7, **gekennzeichnet durch** zusätzliche Vitamine und/oder Mineralien und/oder Spurenelemente und/oder Provitamine.

9. Präparat nach einem der Ansprüche 6 bis 8, **gekennzeichnet durch** zusätzliche pharmakologische Wirkstoffe.

10. Präparat nach einem der Ansprüche 6 bis 9, **dadurch gekennzeichnet, dass** es eine der folgenden Pflanzengattungen enthält:
| | |
|---|---|
| Acerola | Malpighia punicifolia |
| Artischocke | Cynara scolymus |
| Bärlauch | Allium ursinum |
| Baldrian | Valeriana officinalis |
| Birke | Betula Arten i.B. B. pendula B. pubescens |
| Bohne | Phaseolus vulgaris |
| Brennessel | Urtica dioica |
| Brunnenkresse | Nasthurtium officinale |
| Fenchel | Foeniculum vulgare |
| Gänsefingerkraut | Potentilla anserina |
| Ginkgo | Ginkgo biloba |
| Ginseng | Panax ginseng C.A. Meyer |
| Hafer | Avena sativa |
| Huflattich | Tussilago farfara |
| Johanniskraut | Hypericum perforatum |
| Kamille | Matricaria chamomilla |
| Knoblauch | Allium sativum |
| Kürbis | Cucurbita Arten i.B. C. pepo varietäten |
| Löwenzahn | Taraxacum officinale |
| Meerrettich | Armoracia rusticana |
| Melisse | Melissa officinalis |
| Mistel | Viscum album |
| Petersilie | Petroselium sativum |
| Rosmarin | Rosmarinus officinalis |
| Salbei | Salvia officinalis |
| Schafgarbe | Achillea millefolium |
| Schwarzrettich | Raphanus sativus varietäten |
| Sellerie | Apium graveolens |
| Sonnenhut | Echinacea angustifolia/purpurea |
| Spargel | Asparagus officinalis |
| Spitzwegerich | Plantago lanceolata |
| Teufelskralle | Harpagophytum procumbens |
| Thymian | Thymus vulgaris |
| Wacholder | Juniperus communis |
| Weissdorn | Crataegus laevigata/oxyacantha |
| Wermut | Artemisia absinthium |
| Zinnkraut | Equisetum arvense |

## Claims

1. A method for manufacturing a storable preparation from fresh plants, **characterised in that**
- from freshly harvested plant parts by way of pressing, fresh plant juice is extracted which is subjected to a short-time heating to destroy the enzyme activity,
- that up to 20% monosaccharides and/or disaccharides are added to the fresh plant juice for matrix formation,
- that the fresh plant juice is subsequently dried and reduced to powder,
- and that the fresh plant juice powder in a hydrophobic matrix is encapsulated in capsules, in particular soft gelatine capsules.

2. A method according to claim 1, **characterised in that** the freshly harvested plant parts are broken up by vacuum extrusion.

3. A method according to claim 1 or 2, **characterised in that** rosemary extract is added to the fresh plant juice as an antioxidant.

4. A method according to one of the claims 1 to 3, **characterised in that** the drying of the fresh plant juice is effected with the vacuum belt drying method at less than 50° C temperature and at 6 to 15 mbar pressure.

5. A method according to one of claims 1 to 4, **characterised in that** as a hydrophobic matrix long-chained triglycerides and the addition of waxes is used.

6. A storable preparation from fresh plants, in particular manufactured according to the method according to claim 1, **characterised by** a soft gelatine capsule with a filling of fresh plant powder which is embedded into a hydrophobic matrix.

7. A preparation according to claim 1, **characterised by** additional flavour and odour correctors.

8. A preparation according to claim 6 or 7, **characterised by** additional vitamins and/or minerals and/or trace elements and/or provitamins.

9. A preparation according to one of claims 6 to 8, **characterised by** additional pharmacological active ingredients.

10. A preparation according to one of claims 6 to 9, **characterised in that** it contains one of the following plant species:
| | |
|---|---|
| acerola | Malpighia punicifolia |
| artichoke | Cynara scolymus |
| Bärlauch | Allium ursinum |
| valerian | Valeriana officinalis |
| birch | Betula types in particular |
| | Betula pendula |
| | Betula pubescens |
| bean | Phaseolus vulgaris |
| nettle | Urtica dioica |
| watercress | Nasthurtium officinale |
| fennel | Foeniculum vulgare |
| daisy potentilla | Potentilla anserina |
| ginkgo | Ginkgo biloba |
| gingseng | Panax ginseng C. A. Meyer |
| oats | Avena sativa |
| coltsfoot | Tussilago farfara |
| Saint John's wort | Hypericum perforatum |
| camomile | Matricaria chamomilla |
| garlic | Allium sativum |
| gourds | Cucurbita types, in particular |
| | Cucurbita pepo varieties |
| dandelion | Taraxacum officinale |
| horseradish | Armoracia rusticana |
| balm | Melissa officinalis |
| mistletoe | Viscum album |
| parsely | Petroselium sativum |
| rosemary | Rosmarinus officinalis |
| sage | Salvia officinalis |
| yarrow | Achillea millefolium |
| black radish | Raphanus sativus varieties |
| celery | Apium graveolens |
| cone flower | Echinacea angustifolia/purpurea |
| asparagus | Asparagus officinalis |
| ribwort | Plantago lanceolata |
| rampion | Harpagophytum procumbens |
| thyme | Thymus vulgaris |
| juniper | Juniperus communis |
| hawthorn | Crataegus laevigata/oxyacantha |
| wormwood | Artemisia absinthium |
| horsetail | Equisetum arvense |

## Revendications

1. Procédé pour fabriquer une préparation à base de plantes apte à être conservée, **caractérisé**
- **en ce qu'**on extrait de parties de plantes fraîchement cueillies, par pressage, un jus de plante fraîche qui est soumis à un bref réchauffement pour détruire l'activité enzymatique,
- **en ce qu'**on ajoute jusqu'à 20 % de monosaccharides et/ou de disaccharides au jus de plante fraîche, pour former une matrice,
- **en ce qu'**on soumet ensuite le jus de plante fraîche à un séchage et à une pulvérisation,
- et **en ce qu'**on met la poudre issue du jus de plante fraîche, dans une matrice hydrophobe, en capsules et en particulier en capsules de gélatine molle.

2. Procédé selon la revendication 1, **caractérisé en ce que** les parties de plantes fraîchement cueillies sont décomposées par extrusion sous vide.

3. Procédé selon la revendication 1 ou 2, **caractérisé en ce qu'**on ajoute au jus de plante fraîche, comme agent antioxydant, de l'extrait de romarin.

4. Procédé selon l'une des revendications 1 à 3, **caractérisé en ce que** le séchage du jus de plante fraîche se fait selon le procédé du séchage à bande sous vide, à une température inférieure à 50°C et avec une pression de 6 à 15 mbar.

5. Procédé selon l'une des revendications 1 à 4, **caractérisé en ce qu'**on utilise comme matrice hydrophobe des triglycérides à longue chaîne avec une adjonction de cires.

6. Préparation à base de plantes fraîches apte à être conservée, fabriquée en particulier à l'aide du procédé selon la revendication 1, **caractérisée par** une capsule de gélatine molle avec un remplissage de jus de plante fraîche en poudre qui est intégré dans une matrice hydrophobe.

7. Préparation selon la revendication 6, **caractérisée en ce qu'**elle comprend en outre des correctifs de goût et d'odeur.

8. Préparation selon la revendication 6 ou 7, **caractérisée en ce qu'**elle comprend en outre des vitamines et/ou des minéraux et/ou des éléments à l'état de traces et/ou des provitamines.

9. Préparation selon l'une des revendications 6 à 8, **caractérisée en ce qu'**elle comprend en outre des substances actives pharmacologiques.

10. Préparation selon l'une des revendications 6 à 9, **caractérisée en ce qu'**elle contient l'une des espèces de plantes suivantes :
| | |
|---|---|
| acerola | Malpighia punicifolia |
| artichaut | Cynara scolymus |
| ail d'ours | Allium ursinum |
| valériane | Valeriana officinalis |
| bouleau | genres Betula, notamment B. pendula B. pubescens |
| haricot | Phaseolus vulgaris |
| ortie | Urtica dioica |
| cresson de fontaine | Nasthurtium officinale |
| fenouil | Foeniculum vulgare |
| patte d'oie | Potentilla anserina |
| ginkgo | Ginkgo biloba |
| ginseng | Panax ginseng C.A. Meyer |
| avoine | Avena sativa |
| tussilage | Tussilago farfara |
| millepertuis | Hypericum perforatum |
| camomille | Matricaria chamomilla |
| ail | Allium sativum |
| citrouille | genres cucurbita, notamment variétés C. pepo |
| pissenlit | Taraxacum officinale |
| raifort | Armoracia rusticana |
| mélisse | Melissa officinalis |
| gui | Viscum album |
| persil | Petroselium sativum |
| romarin | Rosmarinus officinalis |
| sauge | Salvia officinalis |
| achillée | Achillea millefolium |
| radis noir | variétés Raphanus sativus |
| céleri | Apium graveolens |
| rudbeckia | Echinacea angustifolia/purpurea |
| asperge | Asparagus officinalis |
| plantain lancéolé | Plantago lanceolata |
| harpagophytum | Harpagophytum procumbens |
| thym | Thymus vulgaris |
| genévrier | Juniperus communis |
| aubépine | Crataegus laevigata/oxyacantha |
| absinthe | Artemisia absinthium |
| prêle des champs | Equisetum arvense. |
